(19) Europäisches Patentamt
European Patent Office
Office européen des brevets

(11) Publication number: 0 080 281
A1

(12) # EUROPEAN PATENT APPLICATION

(21) Application number: 82305875.5

(22) Date of filing: 04.11.82

(51) Int. Cl.³: C 07 D 233/78, C 07 D 233/72, A 61 K 31/415

(30) Priority: 04.11.81 GB 8133259
26.11.81 GB 8135667

(43) Date of publication of application: 01.06.83
Bulletin 83/22

(84) Designated Contracting States: AT BE CH DE FR GB IT LI LU NL SE

(71) Applicant: WIGGLESWORTH LIMITED, Peel Street, Westhoughton Bolton, BL5 3SL (GB)

(72) Inventor: Harrison, Anthony Ralph, 6 Greenvale Shevington, Wigan Lancashire (GB)
Inventor: Morrison, Alexander McKenzie, 554 Bolton Road, Bury Lancashire (GB)

(74) Representative: Dayneswood, Trevor et al, ABEL & IMRAY Northumberland House 303-306 High Holborn, London, WC1V 7LH (GB)

(54) **Therapeutic use of allantoin-formaldehyde condensation products.**

(57) Monomeric, dimeric and polymeric allantoinformaldehyde condensation products, especially imidazolidinyl urea and diazolidinyl urea, have been found to be therapeutically effective in the treatment of humans and animals, especially in the treatment of diseases and malconditions of the skin and other membranes. The compounds have the advantages of water-solubility, low toxicity, low irritancy and broad action, which is a combination of properties not found in many drugs. The compounds may be used in a wide variety of compositions and formulations.

EP 0 080 281 A1

"Therapeutic use of allantoin-formaldehyde condensation products"

The present invention relates to the therapeutic use of certain allantoin-formaldehyde condensation products, and to therapeutic compositions containing such products.

Various "monomeric, dimer and polymeric condensation products of formaldehyde and allantoin in the proportions of 1 mole of allantoin to at least about 0.5 mole of formaldehyde", and their salts with acids and bases, have been described in U.S. Patent 3 248 285 (issued 26 April 1966 to P.A. Berke, assignor to Sutton Laboratories, Inc.).

The "monomeric condensation products" have the formula

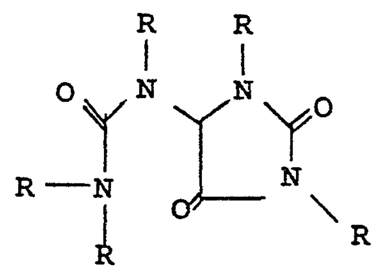

"wherein each R is selected from the group consisting of H and $CH_2OH$, all R's not being simultaneously hydrogen and at least one R being $CH_2OH$". In the dimer condensation products, the monomeric units have the formula given above "wherein each R is selected from the group consisting of H, $CH_2OH$ and $>CH_2$, all R's not being simultaneously hydrogen and at least one R being $>CH_2$ which constitutes a methylene bridge to a nitrogen atom of another monomeric unit". In the polymeric condensation products, the monomeric units have the formula given above "wherein each R is selected from

the group consisting of H, $CH_2OH$ and $>CH_2$, all R's not being simultaneously hydrogen and at least two R's being $>CH_2$ constituting methylene bridges to corresponding and non-corresponding nitrogen atoms of additional monomeric units".

The condensation products are described as being capable of forming "solutions of varying or predetermined viscosity" and also of forming "clear, transparent, hard and glossy films" and, because of those properties, being suitable for use in "a number of plastic, pharmaceutical, cosmetic and industrial formulations". The products are also described as having "bacteriostatic and/or bactericidal properties", rendering them "useful in the preservation of water and food" and also "useful for agriculture and in the paint, plastic and textile industries".

One such dimer condensation product that has become commerically available is the compound known by the common name "imidazolidinyl urea" and also by the trade name "Germall 115" ("Germall" is a Trade Mark). That compound is methanebis[N,N'-(5-ureido-2,4-diketotetrahydroimidazole)N,N-dimethylol, also known as N,N'-methylenebis[N'-[1-(hydroxymethyl)-2,5-dioxo-4-imidazolidinyl]urea], and has the formula

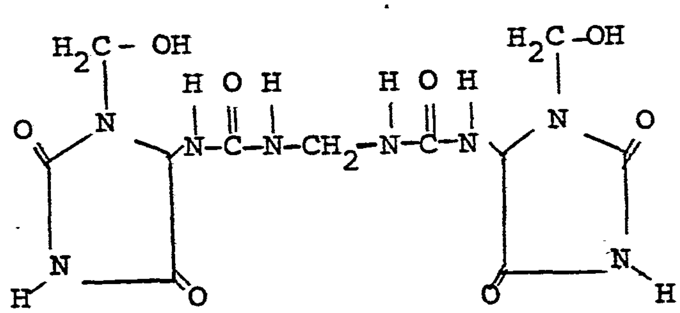

It has been widely used as an antimicrobial preservative in various cosmetic formulations, as described, inter alia, by P.A. Berke and W.E. Rosen in "Germall, a new family of antimicrobial preservatives for cosmetics", American Perfumer and Cosmetics 85(3), 55-59 (March 1970); by Rosen and Berke in "Germall 115 - a safe and effective modern cosmetic preservative", Cosmetics and Toiletries 92(3), 88-89 (March 1977); by Berke and Rosen in "Imidazolidinyl urea activity against Pseudomonas", Journal of the Society of Cosmetic Chemists 29, 757-766 (December 1978); by Rosen, Berke, T. Matzin and A.F. Peterson in "Preservation of cosmetic lotions with imidazolidinyl urea plus parabens", ibid, 28, 83-87 (February 1977); by Berke and Rosen in "Are cosmetic emulsions adequately preserved against Pseudomonas?", ibid, 31, 37-40 (January/February 1980); in "Final report of the safety assessment for imidazolidinyl urea" (by the Cosmetic Ingredient Review Expert Panel of the Cosmetic, Toiletry and Fragrance Association, Inc., U.S.A.), Journal of Environmental Pathology and Toxicology 4(4), 133-146 (October 1980); in US 4 268 502 (issued 19 May 1981 to J.O. Martin, assignor to Eli Lilly & Company); in US 4 272 519 (issued 9 June 1981 to A.M. Herrold et al, assignors to Eli Lilly & Co.); in US 4 272 544 (issued 9 June 1981 to J.A. Cella et al, assignors to Eli Lilly & Co.); in US 4 278 570 (issued 14 July 1981 to M.G. Flom, assignor to Eli Lilly & Co.); in US 4 216 201 (issued 5 August 1980 to L.C. Calvo, assignor to Germaine Monteil Cosmetiques Corp.); and in DE 2 304 156 A (published 1 August 1974, Beecham Inc.).

A monomeric condensation product of the type mentioned above that has more recently become commerically available is the compound known by the common name "diazolidinyl urea" and also by the trade name "Germall II". That compound is

N-(hydroxymethyl)-N-(1,3-dihydroxymethyl-2,5-dioxo-4-imidazolidinyl)-N'-hydroxymethyl urea, and has the formula

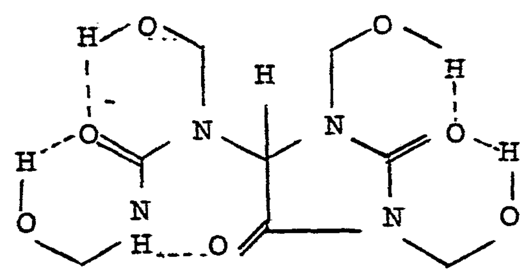

It is described in US 4 271 176 (issued 2 June 1981 to Berke & Rosen, assignors to Sutton Laboratories, Inc.) (corresponding to WO 81/00566, published 5 March 1981, and EP 0 032 500).

Imidazolidinyl urea has been used for some time as a preservative in a wide variety of cosmetic and toiletry products, and much work has been published regarding its antibacterial properties in that respect, both alone and in conjunction with other preservatives. Diazolidinyl urea has been described for use as an antibacterial and antifungal preservative in various cosmetic and toiletry products, especially in creams and lotions, such as shampoos, in a manner similar to that described for imidazolidinyl urea.

It has now been found that imidazolidinyl urea, diazolidinyl urea and related allantoin-formaldehyde condensation products are also useful in the therapeutic treatment of humans and animals. This is particularly surprising since it is some considerable time since these condensation products were first described, and imidazolidinyl urea in particular has been commercially available for a number of years and has been widely

used in cosmetic and toiletry products. Yet there has not previously been any suggestion that the antimicrobial activity of these condensation products could be useful beyond the field of the preservation of cosmetic and toiletry products. In particular, there has not previously been any suggestion that these condensation products could be useful in the therapeutic treatment of humans and animals. Moreover, very many compounds are used as preservatives in cosmetic and toiletry products but their antimicrobial action in that respect does not necessarily suit them for use for therapeutic purposes. Indeed, very many cosmetic preservatives are unsuitable for therapeutic purposes.

The present invention accordingly now provides a monomeric, dimeric or polymeric compound that is a condensation product of formaldehyde and allantoin in a molar proportion of 1 to at least 0.5, respectively, in which the monomeric compound or each monomeric unit is of the general formula

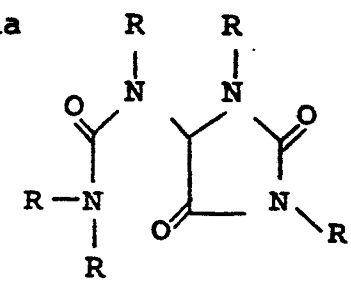

in which each R denotes H, $-CH_2OH$ or $(-CH_2-)_{1/2}$, with the provisos that

(a) in a monomeric compound, R cannot denote $(-CH_2-)_{1/2}$ and at least one R denotes $-CH_2OH$,

(b) in each unit of a dimeric compound and in each terminal unit of a polymeric compound, at least one symbol R denotes $(-CH_2-)_{1/2}$,

(c) in each non-terminal unit of a polymeric compound, each of at least two symbols R denotes $(-CH_2-)_{1/2}$.

or a physiologically tolerable salt of such a compound, for use in the therapeutic treatment of humans or animals.

The present invention also provides a composition which comprises a therapeutically effective amount of a monomeric, dimeric or polymeric compound of the type defined above, or a physiologically tolerable salt of such a compound, in admixture or conjunction with a pharmaceutically or veterinarily suitable carrier.

The said compound is preferably imidazolidinyl urea or diazolidinyl urea.

The compounds used according to the invention (which are also referred to herein as "the allantoin-formaldehyde condensation products" and as "the condensation products") and their physiologically tolerable salts may be used against a wide range of diseases and disorders of the human or animal body. They find particularly efficacious use as antimicrobial agents in the therapeutic treatment of humans and animals, but there are also instances where their therapeutic efficacy cannot be attributed simply to antimicrobial action. Moreover, it seems that, in many cases, the compounds and their physiologically tolerable salts show a potentiating effect when used in combination with known drugs. The compounds and the salts are especially useful in the therapeutic treatment of diseases, malconditions and other disorders of the skin and other membranes of the human or animal body.

(The terms "compound" and "condensation product", and like terms, as used hereinafter include the said physiologically tolerable salts unless the context requires otherwise.)

The minimum therapeutically effective amount

of a compound used according to the invention will depend on a number of factors, not least of which is the type of composition in which it is incorporated. In all cases, however, the minimum therapeutically effective amount will be a concentration that is greater than that which would be required if the compound were present in the composition merely as a preservative to prevent bacterial or fungal growth in the composition. The therapeutically effective amount is generally more than 1% by weight and, at least in some compositions, is advantageously at least 1.5% by weight; there are applications for which the concentration of the compound in the composition is advantageously at least 2% by weight, preferably at least 5% by weight; all percentages being based on the total weight of the composition.

The antimicrobial properties of imidazolidinyl urea are primarily antibacterial (that is to say, both bactericidal and bacteriostatic) properties, whereas the antimicrobial properties of diazolidinyl urea include both antibacterial properties and also antifungal properties (that is to say, activity against yeasts, moulds and other fungal microorganisms).

Diazolidinyl urea is in general effective against all microorganisms against which imidazolidinyl urea is effective, and its activity is generally greater than that of imidazolidinyl urea. Diazolidinyl urea has, for example, been found to be effective, at a concentration of 0.2%, against gram-negative bacteria such as Pseudomonas aeruginosa (ATCC 9027) and Escherichia coli (ATCC 10536), and also against P. cepacia at a concentration of 0.5% or at a concentration of 0.3% in

combination with Parabens. In fact, diazolidinyl urea is about 20% more effective against Pseudomonas, which is a pathogen that it is important to be able to combat, than is imidazolidinyl urea. Moreover, diazolidinyl urea has also been found to be effective against yeasts, moulds and other fungi: it has, for example, been found to be effective against A. niger (ATCC 16404) at a concentration as low as 0.1% and against C. albicans (ATCC 10231) at a concentration of 0.3 to 0.4%.

The antimicrobial properties of these compounds may be used against a wide range of pathogens associated with diseases and other disorders in both humans and animals. In particular, they are useful for the non-antibiotic chemotherapeutic treatment of both humans and animals for both prophylactic (that is to say, preventive) and curative purposes. (The term "therapeutic treatment" and like terms as used herein include both prophylactic treatment and curative treatment, unless the context requires otherwise.)

These compounds are particularly useful in siutations where antibiotics (especially β-lactam antibiotics, such as penicillins and cephalosporins cannot, or cannot effectively, be used, and also in situations where the use of antibiotics would be undesirable. They can, for example, be used for the treatment of pathogens that have become resistant to certain antibiotics. Moreover, because of their low toxicity, they can also be used in higher concentrations than those in which antibiotics can be used, and because of their low irritancy they can also be used in higher concentrations than those in which many other non-antibiotic chemotherapeutic substances can be used.

Various properties of the compounds, in addition to their activity against a large number of microorganisms, render them suitable for a wide variety of veterinary, pharmaceutical and like applications. As mentioned above, the compounds have a low toxicity, which not only means that they can be used in higher concentrations than can more toxic drugs, but also means that they can be administered more freely and more safely, without any substantial risk of adverse effects caused by inadvertant overdosage or of side effects. Also, the risks of environmental pollution are substantially reduced. Diazolidinyl urea is more toxic than is imidazolidinyl urea, but its toxicity is still at a very low level.

The compounds also have a low irritancy, so that they can broadly be described as non-irritant. Indeed, in some cases, the compounds show an anti-irritant effect, that is to say that, not only do they cause no irritancy themselves, but they can in fact reduce irritancy caused by other substances. The low irritancy of the compounds again enables them to be used in higher concentrations than can many other drugs, and also enables them to be used on highly sensitive tissue, for example on burns and lacerations, in the eye, on cattle udders, and internally.

Many of the compounds are water-soluble and can therefore be formulated more easily than can water-insoluble drugs and they can be used for purposes for which water-insoluble drugs are not suitable. The water solubility of those compounds also enables them to be more readily absorbed into the body.

The compounds also have film-forming properties, which are useful for certain applications, especially where the drug is to be applied externally and might otherwise readily become removed more quickly than desired.

In the veterinary field, the compounds are particularly useful in the prevention or cure of

mastitis, primarily in cattle. They are also useful in the treatment of the intestines to counteract various enteric infections, for example neo-natal scour and gastro-enteritis in various animals including, for example, cattle, lambs, foals, pigs, poultry, fish, and exotic animals; in the treatment of the uterus to counteract uterine infections, such as acute endometritis, which can occur in many animals, especially horses; in the treatment of animal hooves, to counteract foul-in-the-foot and other types of foot rot; in the treatment of the ears and eyes of animals; and also in the treatment of fish, particularly in fish farming, for example be being incorporated in a fish feed or by being used in a treatment tank.

In the pharmaceutical field, these compounds are particularly useful in the treatment of diseases, malconditions and other disorders of the skin, including, especially, ammoniacal dermatitis (in the form of nappy rash and bed sores, for example) and also acne, ulcers, and other dermatological conditions. They are also useful in the chemotherapeutic treatment of burns, skin grafts and skin lacerations of the human body. Furthermore, they may be used in a mouthwash for, for example, pre-dental or post-dental treatment; and in the treatment of ears and eyes of humans and in the treatment of contact lenses, for example in eye drops and in solutions for wetting, cleaning or storing contact lenses (whether hard or soft lenses).

The compounds may also be used for various surgical and like purposes. The compounds may, for example, be applied to or incorporated in - for

example, by impregnation and, for example, in the form of a powder or in the form of a solution - surgical dressing (including plasters, lint, and other wound dressings of any nature whether intended for use in a clinical, domestic, industrial or other situation), surgical swabs (for example, pre-operative swabs) and the like. Solutions of the compounds, optionally containing other substances, may be used for treating surgical instruments, surgical apparatus and the like, for cleaning and sterilisation purposes, and also for treating articles used for preparing, containing or administering food, especially baby food. The compounds may be used in a peritoneal or other lavage and in an enema, for example for intestinal sterilisation prior to surgical operations on the large or small intestines. The compounds may also be incorporated in surgical soap or the like used by surgeons and their assistants in "scrubbing-up", in an amount sufficient to be effective in aiding sterilisation of the surgeon's or assistant's hands.

The compounds may furthermore be used in the treatment of milk for preservation purposes.

The compounds may be formulated for pharmaceutical or veterinary use, that is to say, for the therapeutic treatment of the human or animal body, in a variety of forms depending on the particular intended use. They may be incorporated in pharmaceutical, veterinary or like compositions, in admixture or conjunction with a carrier (which term, as used herein, includes an excipient). The composition may, for example, be formulated for external application to humans or animals or for internal application to humans or animals. The compositions may be formulated in unit-dosage form.

The carrier incorporated in the composition should be a pharmaceutically or veterinarily suitable carrier. The carrier may comprise one or more carriers selected from (a) a non-liquid carrier, optionally in conjunction with a liquid carrier, (b) a liquid carrier other than water, optionally in conjunction with water, and (c) water in conjunction with another substance. For a number of purposes the carrier may comprise physiologically tolerable saline solution.

The compositions according to the invention may be formulated in a wide variety of forms, using a wide variety of carriers and auxiliaries.

Examples of types of formulation suitable for the treatment of animals include compositions for parenteral or external application to cattle udders, for example in the form of a gel or jelly, or in the form of a solution or other liquid; compositions for enteral administration to animals, for example a bolus (for use with larger animals, for example calves, sheep and other ruminants) or a capsule or tablet (for use with smaller animals, for example cats and dogs); formulation as a uterine infusion; compositions for external application to animal hooves, for example as a spray, especially a water-based spray; compositions for external application to the ears or eyes of animals, for example as an ointment or as ear or eye drops; and formulation as an aqueous solution, for a variety of applications.

Examples of types of formulation suitable for the treatment of humans include various preparations for application to the skin of humans, for example creams, jellies, gels, ointments, sprays

and the like; as well as preparations for external application to the ears or eyes of humans, for example ointments and ear and eye drops; formulation as a mouthwash; and formulation as capsules, tablets, pills and the like for enteral administration.

Compositions according to the invention for the therapeutic treatment of animals may contain the active ingredient in concentrations of anything up to 100%, depending on the intended use of the particular formulation, but advantageously concentrations of up to 50% by weight, preferably up to 20% by weight, based on the total weight of the composition, are used.

Compositions according to the invention for the therapeutic treatment of humans may contain the active ingredient in concentrations of up to 30% by weight, although for some purposes the concentration advantageously does not exceed 20% by weight, and for some purposes preferably does not exceed 10% by weight, in each case based on the total weight of the composition.

The compositions according to the invention may contain other therapeutically active ingredients (for example, topical steroids, antibiotics, and other antimicrobial agents), as well as topical anaesthetics, preservatives, emulsifiers, dispersants, propellants, thickening agents, colouring agents, buffers, flavouring agents, sweetening agents, carriers (including excipients, solvents, and solid and liquid diluents), binders, disintegrants, lubricants, and other conventional auxiliaries. The choice of auxiliaries for any particular composition will, of course, be affected by the type of composition, whether the composition is intended for internal or external application, and whether it is intended for animal or human use. The auxiliaries used should, of course, normally be

physiologically tolerable, especially when the composition is intended for internal application. The compositions according to the invention may be prepared in a conventional manner known to those skilled in the art.

It is often advantageous for the compositions according to the invention additionally to comprise esters of para-hydroxybenzoic acid, preferably alkyl para-hydroxybenzoates, especially $C_1$-$C_4$ alkyl para-hydroxybenzoates (which are commonly known as the "Parabens" - "Paraben" is a registered Trade Mark), for example, methyl or propyl hydroxybenzoate. Such additives tend to improve the activity of the compositions: they can have a synergistic or potentiating effect in conjunction with the allantoin-formaldehyde condensation products used according to the invention. Such additives are preferably used in amounts of at least 2% by weight, based on the weight of the allantoin-formaldehyde condensation product present in the particular composition. They may typically be present in the compositions in amounts of from 0.01 to 5% by weight, based on the total weight of the respective composition. Depending on the nature and intended use of the particular composition, it can be advantageous to ensure that those additives are not present in an amount exceeding their solubility in the particular composition in question.

It is also often advantageous for the compositions according to the invention additionally to comprise soluble allantoin, because of its therapeutic properties.

One important veterinary application of the allantoin-formaldehyde condensation products used according to the invention is their use for the treatment or prevention of mastitis, especially in cattle. The udder tissue of cattle provides a very

good breeding ground for a wide variety of both gram negative and gram positive pathogens including, for example, Staphylococcus aureus, Streptococcus bovis, Str. agalactia, Str. disgalactia, Str. uberis, Escherichia coli, Corynebactrium pyogenes, and Pseudomonas aeruginosa. The udder tissue is very sensitive and it is therefore important that any formulations or other preparations used for treating the udder should have a low irritancy. The treatment of mastitis in cattle has hitherto generally been carried out using antibiotics, but their prolonged use can create problems of the pathogens' developing resistance to the antibiotics used, and many of the pathogens mentioned above are now considered to be penicillin-resistant, ss a result of over-exposure to sub-lethal doses of various antibiotics. Moreover, the use of antibiotics on lactating cows can create public health problems, and for antibiotics to be fully effective clinical typing is necessary.

Mammary products based on the allantoin-formaldehyde condensation products used according to the invention can be used for the non-antibiotic chemotherapeutic treatment of mastitis without many of the disadvantages associated with the use of antibiotics. These products have a bactericidal action against many of the pathogens associated with mastitis, of both gram-negative and gram-positive types, and including penicillin-resistant strains. They could, therefore, be effective in overcoming a situation where a disease had become endemic. These products also have several other advantages rendering them very suitable for this purpose: they have a very low degree of irritancy in the udder; they

have a very low toxicity, which means that they can be used in suckled cows without danger to the calves and also that there is no danger to human consumers of milk from treated cows; they are water-soluble and are therefore effective when used in a medium such as milk; and they are not inhibited by the presence of proteins such as are present in milk - on the contrary, there is a potentiating effect, which can be valuable.

Compositions according to the invention intended for the treatment of lactating cows may suitably be formulated as an intramammary product for parenteral application, for example in the form of a gel or the like, especially a thin gel that can be injected into the teat. A composition according to the invention intended for intramammary use may contain from 1 to 10% by weight, preferably up to 7.5% by weight, and especially from 1 to 2% by weight, of the allantoin-formaldehyde condensation product, based on the total weight of the composition. The greater is the concentration used, the longer is the retention time of the active ingredient in the milk. The milk would not normally be used for human consumption while it still retains some active ingredient and it is therefore advantageous that the retention time should not be too long. With an active ingredient content in the composition of about 1 to 2%, the milk may be free of active ingredient after as few as two milkings, but in any case milk from the initial milkings can be fed to calves, whereas when using intramammary products containing antibiotics the milk would have had to be wasted. The retention time of imidazolidinyl urea is lower than that of diazolidinyl urea, but the efficacy of the former compound

is also low. It can be advantageous to use a mixture of those two compounds as the active ingredient.

A composition formulated for intramammary use of a lactating cow may suitably comprise from 1 to 7.5% by weight of imidazolidinyl urea and/or diazolidinyl urea; from 10 to 25% by weight of a carrier, preferably a water-soluble or water-miscible carrier, for example glycerine, propylene glycol, sorbitol, or another polyhydric alcohol; from 0.1 to 1% of benzoic acid or a hydroxybenzoate, preferably in a concentration not exceeding its solubility in water; from 0.1 to 7.5% of a gelling or thickening agent, for example, sodium carboxymethylcellulose, starch, or xanthan gum; and water to 100%; all percentages being based on the total weight of the composition.

Compositions according to the invention intended for the mammary treatment of a dry cow may be formulated in a similar manner to the intramammary product described above but with a higher proportion of gelling or thickening agent to give a more viscous product. Such a product may be injected into the mouth of the teat where, on exposure to air, it will dry to form a rubbery product that will block the teat canal, thus preventing the ingress of infection from flies, for example.

Alternatively, for use on a dry cow, a composition may be formulated as a gel, jelly, cream, ointment, wax or the like for extramammary use. An oily - as distinct from aqueous - composition may be prepared for use on a dry cow so that it will not easily be washed away.

Compositions for use on a dry cow may contain up to 10% by weight, based on the total weight of the composition, of the allantoin-formaldehyde condensation product, and preferably contain at least 5% by weight thereof. They may also contain allantoin to assist

in healing any damage to the udder. A composition formulated for extramammary use on a dry cow may suitably comprise from 5 to 10% by weight of imidazolidinyl urea and/or diazolidinyl urea; up to 95% by weight of a carrier, for example from 50 to 75% by weight of aluminium stearate and from 10 to 35% by weight of liquid paraffin or of a mixture of glycerine and up to 5% by weight of sodium carboxymethyl cellulose; up to 10% by weight of an emulsifier, for example a polysorbate, such as "Tween 80" ("Tween" is a registered Trade Mark); and from 0.05 to 4% by weight of a hydroxybenzoate; all percentages being based on the total weight of the composition.

Compositions according to the invention may also be formulated as an aqueous solution for use as an udder wash and for washing teat tips.

Another veterinary application of the allantoin-formaldehyde condensation products is their use in the treatment of enteric infections, for example neo-natal scour and gastro-enteritis in calves, lambs, foals, and other animals. Pathogens associated with these conditions include Escherichia coli and the Salmonella group, both of which inhabit the intestines and, under disease conditions, can cause severe damage or even death. These conditions have previously been treated with antibiotics, sulphonamide drugs, and chloramphenicol. The use of chloramphenicol for this purpose is, however, now banned in the United Kingdom, and the pathogens have developed strains that are resistant to antibiotics and even to trimethoprim/sulphonamide products.

The low irritancy and toxicity of the allantoin-formaldehyde products mean that they can safely be given to animals in sufficiently large doses for bactericidal effectiveness in enteral use. Because they are water-soluble, they will diffuse through the contents of the gut quickly, thus improving their effectiveness. They have been found to be effective against both types of pathogens mentioned above, including penicillin-resistant strains, and, morever, as mentioned previously, there is a potentiating effect in the presence of proteins, which usually constitute an important component in the diet of young animals.

Diazolidinyl urea intended for the treatment of enteric infections may be formulated as oral preparations, such as a bolus (for use with large animals) and capsules, pills or tablets (for use with smaller animals).

Such compositions in the form of a bolus, capsules, pills, tablets and the like may contain the allantoin-formaldehyde products in concentrations of up to 15% by weight, especially from 2.5 to 15% by weight, based on the total weight of the composition (excluding, in the case of capsules, the shell of the capsule), in admixture or conjunction with excipients, bulking agents, lubricating agents, disintegrating agents, binding agents and other conventional auxiliaries. Capsules, pills and tablets may, for example, be prepared in two sizes: a capsule of which the contents is up to 0.8 g, or a pill or tablet of up to 0.8 g, for use with small animals, for example cats and small dogs; and a capsule of which the contents is up to 2 g, or a pill or tablet of up to 2 g, for use with slightly larger animals, for example large dogs. Boluses may be 5 to 6 g in weight.

A composition in the form of a bolus may suitably comprise from 5 to 15% by weight of imidazolidinyl urea and/or diazolidinyl urea, in admixture with excipients, for example from 5 to 15% of microcrystalline cellulose, from 10 to 25% of starch, from 40 to 80% of lactose, and from 0.1 to 2% of magnesium stearate, with a colouring agent as required, all percentages being based on the total weight of the composition. The bolus should preferably be water-swellable and should preferably disintegrate within 0.5 to 1 minute (as measured *in vitro* in accordance with the British Pharmacopoeia).

A composition in the form of a capsule may suitably comprise, as the capsule contents, from 2.5 to 15% by weight of imidazolidinyl urea and/or diazolidinyl urea, in admixture with up to 97.5% by weight of an excipient, for example starch, lactose, calcium phosphate, or cellulose, and up to 2% by weight of a lubricant, for example stearic acid, all percentages being based on the total weight of the capsule contents.

A further veterinary application of the allantoin-formaldehyde products is the treatment of uterine infections, especially acute endometritis. This can occur in most animals, but can be an especial problem in horses. It is caused by the presence in the uterus of pathogens such as *Streptococcus zoeipidemieus*, haemolytic *Escherichia coli*, the *Klebsiella* group, and *Staphyloccocus aureus* all of which have been found to be vulnerable to imidazolidinyl urea. Drugs intended for use as uterine infusions need to be water-soluble, of low irritancy, of low toxicity, and effective in the biological environment of the uterus; the allantoin-formaldehyde products meet all those desiderata. Moreover, because of their low toxicity and low irritancy, those

products can be incorporated in a uterine infusion in a relatively high concentration if that should be necessary. The high degree of efficacy and safety of the allantoin-formaldehyde products make them valuable for use in animals of high commerical value, for example horses.

Compositions according to the invention formulated for uterine infusion may suitably contain the allantoin-formaldehyde condensation products, advantageously in concentrations of up to 10% by weight, preferably from 2 to 10% by weight, especially from 2 to 5% by weight, based on the total weight of the composition, in admixture or conjunction with physiologically tolerable saline solution. Such a composition may, for example, comprise from 2 to 10% by weight of imidazolidinyl urea and/or diazolidinyl urea; from 0.1 to 3% by weight of a hydroxybenzoate; from 2 to 8% of water; a colouring agent as desired; and buffered normal saline to 100% by weight; all percentages being based on the total weight of the composition.

The allantoin-formaldehyde condensation products may also be used in the treatment of foul-in-the-foot, which can be a serious winter problem with both cattle and sheep. This disease is caused by <u>Fusiformis necrophorus</u> and <u>Fusiformis nodosus</u>; it can be specially prevalent in wet muddy conditions and it is easily transmitted. It has in the past been treated with chloramphenicol, but the use of that drug for this purpose is now banned in the United Kingdom. Again the low irritancy and low toxicity of the condensation products means that they can be used in high concentrations when necessary, and their low toxicity also means that there is little risk to humans in the vicinity even

during long treatment sessions. Persistence of a drug is important in any type of treatment of hooves and that is aided by the film-forming properties of the condensation products, used according to the invention.

For use in the treatment of animal hooves, the compositions according to the invention may suitably be formulated for application as a spray, comprising the condensation products in admixture or conjunction with a film-forming agent and a volatile diluent. The composition may, for example, comprise from 5 to 30% by weight of imidazolidinyl urea and/or diazolidinyl urea; from 1 to 5% by weight of a film-forming agent (other than the allantoin-formaldehyde condensation products), for example polyvinyl pyrollidone; from 5 to 15% by weight of an alcohol, for example ethanol; up to 10% by weight of water; and a volatile solvent, for example 2,2-dimethyl-1,3-dioxalane-4-methanol, to 100%; all percentages being based on the total weight of the composition.

An important pharmaceutical application of the allantoin-formaldehyde condensation products is their use in the treatment of dermatological conditions. One dermatological condition where they may be particularly useful is ammoniacal dermatitis. This condition occurs, generally as a result of urinary incontinence, primarily in paediatric and geriatric patients, in the form of nappy rash or bed sores, for example. Urine on the skin is decomposed by bacteria of the <u>Proteus</u> group with the liberation of ammonia, thus resulting in the establishment of a local alkaline environment on the skin. This enables pathogens such as <u>Pseudomonas</u>, <u>Escherichia coli</u>, the <u>Klebsiella</u> group, and the <u>Citrobactic</u> group to thrive, with resulting inflammation of the skin.

Effective treatment of this condition can be difficult. It is necessary to use a substance that is effective against pathogens such as *Pseudomonas*, which can be difficult to combat, and that is effective in both alkaline and acid environments, because the local alkaline environment has to be changed to the natural acid mantle of the skin. It is also important that the substance used should have a low irritancy, in order that the skin should not be irritated further, and that it should have a low toxicity, because in the treatment of conditions of this nature the drug is spread over a large absorptive area. Few materials have previously been found that satisfy all those conditions, and some of the substances currently used for treating ammoniacal dermatitis are not adequately effective against *Pseudomonas*. The present allantoin-formaldehyde condensation products are effective in acid conditions, and have low irritancy and low toxicity, and they may therefore be used against ammoniacal dermatitis.

Another dermatological condition against which the present products may be used is acne, which is a form of pustular dermatitis associated with pathogens of the *Staphylococcus* group, for example. *S. aureus*, *S. albus*, and *S. epidermidis*. Those pathogens have a particular ability to produce mutants and resistant strains, which make it difficult to treat this condition successfully by means of antibiotics. The present products are effective against those pathogens and have the advantages mentioned above in relation to

ammoniacal dermatitis resulting from their low irritancy and low toxicity. Those properties also mean that the compounds may be used in concentrations adequate to counteract both acute and chronic conditions of acne.

The present products may also be used against the skin condition known as ichthyosis, which is very difficult to treat by means of known drugs.

Compositions according to the invention for use in the treatment of dermatological conditions, may be formulated as gels, jellies, creams, ointments or the like, which may contain the active substance in a concentration of at least 1% by weight, advantageously not more than 20% by weight, and preferably from 1 to 10% by weight, based on the total weight of the composition.

A composition according to the invention formulated as a cream for the treatment of dermatological conditions may suitably comprise from 1 to 20% by weight of imidazolidinyl urea and/or diazolidinyl urea; up to 5% by weight of another antibacterial agent, for example a quaternary ammonium derivative, such as cetyl pyridinium chloride; from 5 to 15% by weight of an emulsifier, for example cetostearyl alcohol; from 0.1 to 3% of a hydroxybenzoate; up to 25% of a protective agent, for example silicone fluid; from 15 to 90% by weight of a carrier, for example liquid paraffin, glycerol, or lanolin; and water to 100% by weight, all percentages being based on the total weight of the composition. If the cream is intended for the treatment of acne, the imidazolidinyl urea and/or diazolidinyl urea is preferably present in a concentration of from 1 to 10% by weight, and the

composition may additionally comprise up to 10% by weight of benzoyl peroxide, up to 2% by weight of halquinol, and up to 25% by weight of sulphur, preferably crystalline sulphur.

A composition formulated as a jelly for external human application may suitably comprise from 1 to 10% by weight of imidazolidinyl urea and/or diazolidinyl urea; from 0.1 to 3% by weight of a hydroxybenzoate; from 5 to 30% by weight of a smoothing agent, for example glycerine or polyethylene glycol; from 1 to 10% by weight of a thickening agent, for example sodium carboxymethylcellulose, xanthan gum or "Carbopol" (Trade Mark); up to 5% by weight of a topical anaesthetic, for example lignocaine; and water to 100% by weight; all percentages being based on the total weight of the composition.

A composition formulated as an ointment for external human or animal application may suitably comprise from 1 to 10% by weight of imidazolidinyl urea and/or diazolidinyl urea; from 0.1 to 3% by weight of a hydroxybenzoate; up to 5% by weight of a topical steroid, for example hydrocortisone acetate; up to 10% by weight of an oil, for example cod liver oil; up to 5% by weight of a deodorising agent, for example chlorophyll; up to 98% by weight of a polyhydric alcohol, for example polyethylene glycol, polypropylene glycol or glycerine; up to 2% by weight of an aromatic oil, for example neoroli or abietis oil; and a colouring agent as required; all percentages being based on the total weight of the composition.

The allantoin-formaldehyde condensation products may also be used in the therapeutic treatment of tissue damaged by burns (of first, second or minor degree), lacerations, and the like in both humans and animals. Tissue that has been damaged in that manner is highly

sensitive and any substance used to treat it must therefore have a very low irritancy even at high concentrations. The damaged tissue is liable to invasion by environmental pathogens and can readily absorb the toxins that they produce. One of the most persistent pathogens, and also one of the most difficult to combat, is Pseudomonas. That pathogen cannot easily be controlled by antibiotics but, on the other hand, most non-antibiotic chemotherapeutic agents are too irritant to use on damaged tissue. The allantoin-formaldehyde condensation products used according to the invention are, however, very suitable for this purpose because of their effectiveness against Pseudomonas, combined with their low irritancy. Moreover, because they are water-soluble, they can be used in an aqueous medium that is compatible with tissue fluids.

For use in the therapeutic treatment of damaged skin, compositions according to the invention may be formulated, for example, as a gel, jelly, ointment, or aqueous spray. An aqueous spray is particularly suitable for the treatment of large areas of damaged tissue, especially that damaged by burns, including cases where skin grafting is to be carried out. A gel or jelly is more suitable for the treatment of smaller areas of damaged tissue, especially that damaged by burns; and an ointment is generally suitable for use where the skin has been lacerated. Compositions according to the invention intended for the treatment of burns may advantageously additionally comprise allantoin.

Compositions in the form of a gel, jelly or ointment may suitably be formulated as described above. Compositions in the form of an aqueous spray may comprise the allantoin-formaldehyde condensation product, suitably in an amount of from 1 to 10% by weight, based on the total weight of the formulation,

in admixture or conjunction with physiologically tolerable saline solution. A composition formulated as an aqueous spray may suitably comprise from 1 to 10% by weight of imidazolidinyl urea and/or diazolidinyl urea, from 0.1 to 3% by weight of a hydroxybenzoate, up to 5% by weight of water, and buffered normal saline to 100% by weight, all percentages being based on the total weight of the composition.

The allantoin-formaldehyde condensation products may also be used for the treatment of ear and eye infections in both humans and animals. Compositions according to the invention intended for this purpose may suitably be formulated as an ointment or as drops, advantageously containing the allantoin-formaldehyde condensation products in an amount of from 1 to 10% by weight, based on the total weight of the composition. Such a composition in the form of an ointment may suitably comprise from 1 to 10% by weight of imidazolidinyl urea and/or diazolidinyl urea; from 0.1 to 2% by weight of a hydroxybenzoate; up to 10% by weight of another antibacterial agent, for example sulphacetamide or chloramphenicol; up to 5% by weight of a topical anaesthetic, for example lignocaine; and up to 99% by weight of a carrier, for example from 50 to 65% by weight of a liquid paraffin and from 25 to 35% by weight of white soft paraffin.

The allantoin-formaldehyde condensation products may also be used in a mouthwash, for example, for use before and/or after dental treatment. Compositions according to the invention formulated as a mouthwash may comprise the allantoin-formaldehyde condensation products, advantageously in a concentration of from 1 to 20% by weight, based on the total weight of the composition, in conjunction with one or more auxiliaries selected from a sweetening agent, a flavouring agent,

and a colouring agent. Such a composition may comprise from 2 to 15% by weight of imidazolidinyl urea and/or diazolidinyl urea; from 0.1 to 3% by weight of a hydroxybenzoate; up to 0.5% by weight of a sweetening agent, for example sodium saccharin; up to 5% by weight of a flavouring agent; a colouring agent as required; from 2 to 5% by weight of a thickening agent, for example sodium carboxymethylcellulose; up to 10% by weight of a polyhydric alcohol; and water to 100% by weight; all percentages being based on the total weight of the composition.

Further examples of compositions according to the invention suitable for human use include compositions formulated as antimicrobial capsules for general systemic applications, compositions formulated as a gel or jelly for mouth ulcers and vaginal infections, and compositions formulated as a water-based spray for foot treatment.

A further example of a composition according to the invention is a composition for skin sterilisation comprising from 1 to 10% by weight of imidazolidinyl urea or diazolidinyl urea; from 0.1 to 1% by weight of a hydroxybenzoate; up to 10% by weight of a chlorhexidine salt; from 10 to 75% by weight of an alcohol, for example methanol, ethanol or isopropanol; up to 2% by weight of a surfactant, for example sodium lauryl sulphate; a colouring agent as required; up to 5% by weight of a thickening agent, for example hydroxyethylcellulose, Carbopol, or sodium carboxymethylcellulose; and water to 100% by weight; all percentages being based on the total weight of the composition.

The allantoin-formaldehyde condensation products may be used in the treatment of fish, especially in fish tanks and fish farming. One drug previously used for the therapeutic treatment of fish is trimethoprim, but that substance is water-insoluble and therefore has to be used by being incorporated in the fish feed. Sick fish do not, however, necessarily take the feed and that is not therefore a very satisfactory method of treatment method. Some drugs previously used for the treatment of fish are ineffective or insufficiently effective against Pseudomonas. Moreover, various water-soluble materials that have previously been used for the treatment of fish in, for example, a treatment tank, are toxic and can present an environment hazard when water containing them is discharged as waste. According to the present invention, fish may be treated by, for example, placing them in a treatment tank containing a solution of the allantoin-formaldehyde condensation product.

Compositions according to the invention suitable for the treatment of fish may be in the form of a fish feed, or may comprise a solution of the condensation product and a fish-fungicide.

The allantoin-formaldehyde condensation products have a number of important advantages over substances previously used for the treatment of many of the infections and diseases mentioned. The combination of their low toxicity, low irritancy and wide range of antimicrobial acitivity is particularly important. Various non-antibiotic chemotherapeutic substances already known have a wide range of antimicrobial acitivity, but have the disadvantage of a high irritancy and therefore cannot be used in certain

applications and in relatively high concentrations and/or have a relatively high toxicity, also preventing them from being used in relatively high concentrations. On the other hand, antibiotic substances have a low irritancy, but their action tends to be fairly specific and many pathogens exist in strains that are resistant to antibiotics, and antibiotic substances also have the disadvantage of having to be used in carefully controlled doses.

The present condensation products thus combine the advantages of antibiotic substances with those of non-antibiotic chemotherapeutic substances. They also have the advantages of being soluble in water, a high degree of safety in use, leaving little or no tissue residue, and having few or no side effects.

When used, for example in solutions for treating contact lenses, they have the advantage that unlike known solutions containing quaternary ammonium salts, they are non-irritant; and when used, for example, in "scrubbing-up" by surgeons, they have the advantage that, unlike some substances used for that purpose, they have a very low toxicity.

The following examples illustrate the manufacture of compositions according to the invention formulated for various applications. All percentages given are calculated by weight and are based on the total weight of the composition, unless otherwise stated. Pressures given in the examples are gauge pressures. The term "active substance" as used in the examples, means imidazolidinyl urea or diazolidinyl urea .

Example 1 - Gel for intramammary use on a lactating cow

| | | |
|---|---|---|
| active substance | | 2.00% |
| glycerine | | 25.00% |
| methyl hydroxybenzoate | | 0.05% |
| propyl hydroxybenzoate | | 0.05% |
| sodium carboxymethylcellulose | | 4.50% |
| water | to | 100.00% |

The methyl hydroxybenzoate and the propyl hydroxybenzoate are mixed with cold water and heated until dissolution occurs, and the solution is cooled and filtered. The active substance is dissolved in cold water and the solution is filtered. The two solutions are then mixed, together with further water, and stirred thoroughly. The pH is adjusted to about 6.3 using normal sodium hydroxide solution or 10% hydrochloric acid. The glycerine and sodium carboxymethylcellulose are mixed and stirred to form a smooth paste. The solution is then added to the paste, made up to final volume with water, and stirred thoroughly.

A sample is then autoclaved at about 15 $lb/in^2$ (about 1 bar) for about 40 minutes and then its viscosity is checked. If the viscosity is not within the range of from 8000 to 15000 cP, an additional portion of sodium carboxymethylcellulose is added to a second sample, which is then autoclaved as before, after which its viscosity is checked as before. When an autoclaved sample of the correct viscosity has been obtained, any necessary adjustments

are made to the remaining bulk, and that is then autoclaved for 40 minutes.

The autoclaved product may then be filled into plastic syringes with, for example, a 10 g fill. The final product should preferably have a viscosity of about 3000 cP so that it can flow readily from the syringe.

Example 2 - Ointment for extramammary use on a dry cow

| | |
|---|---|
| active substance | 8.0% |
| aluminium stearate | 65.0% |
| liquid paraffin | 20.0% |
| "Tween 80" | 5.0% |
| methyl hydroxybenzoate | 1.0% |
| propyl hydroxybenzoate | 1.0% |

The aluminium stearate, liquid paraffin and Tween 80 are mixed and heated at 150°C for 1 hour and then allowed to cool to 35 - 40°C. The methyl hydroxybenzoate and propyl hydroxybenzoate are added to the resulting mixture, while being stirred, and the active substance is then added with further stirring, which is then continued for about 15 minutes after all ingredients have been added. The mixture is then passed through a colloid mill, and finally mixed with a high-speed stirrer for about 30 minutes.

Example 3 - Bolus for enteric infections in large animals

| | | |
|---|---|---|
| active substance | | 10.0% |
| microcrystalline cellulose | | 10.4% |
| starch | | 19.5% |
| magnesium stearate | | 0.5% |
| colour | | 0.5% |
| lactose | to | 100.0% |

The bolus may be prepared by direct compression of a mixture of the components listed above, or the components may first be subjected to aqueous or non-aqueous granulation.

Example 4 - Capsules for enteric infections in small animals

| | |
|---|---|
| active substance | 5.00% |
| starch | 94.65% |
| stearic acid | 0.35% |

The percentages given above in this example are based on the capsule contents.

The active substance and the starch are blended in a drum blender, and the compacted under a pressure of about 70 $lb/in^2$ (about 4.75 bar) on a roller compactor operated at slow speed. The resulting graunles are passed through a 1 mm mesh sieve, blended with the stearic acid, and then filled into gelatine shells. Alternatively, the mixture may be filled into the shells as a powder or after aqueous or non-aqueous granulation.

Example 5 - Uterine infusion

| | |
|---|---|
| active substance | 3.00% |
| methyl hydroxybenzoate | 1.00% |
| propyl hydroxybenzoate | 1.00% |
| dye | 0.05% |
| water | 5.00% |
| buffered normal saline | to 100.00% |

The methyl hydroxybenzoate and propyl hydroxybenzoate are added to water heated to about 80°C, and the mixture is stirred until a clear solution is formed. The active substance is dissolved in cold water and the dye is added to the resulting solution. The two solutions are then mixed together, stirred, and made up to final volume with the buffered normal saline. The pH is adjusted to between 6.0 and 6.5 by adding normal sodium hydroxide solution or 10% hydrochloric acid as necessary.

Example 6 - Aqueous spray for treatment of foot rot

| | |
|---|---|
| active substance | 20.0% |
| polyvinyl pyrollidone | 2.0% |
| ethanol | 10.0% |
| water | 10.0% |
| basic fuchsin (colour) | 1.0% |
| 2,2-dimethyl-1,3-dioxalane-4-methanol | to 100.0% |

The ethanol and polyvinyl pyrollidone are mixed, with stirring, until a clear solution is formed. The active substance is dissolved in

cold water, the basic fuchsin is added to the resulting solution, stirring is continued for about 15 minutes, and the mixture is filtered through a sieve. First the active substance solution and then the ethanol solution are added to the solvent, and the final solution is stirred for 15 minutes.

Example 7 - Ointment for ear and eye infections

| | |
|---|---|
| active substance | 2.0% |
| methyl hydroxybenzoate | 0.5% |
| propyl hydroxybenzoate | 0.5% |
| sodium sulphacetamide | 6.0% |
| lignocaine hydrochloride | 0.5% |
| liquid paraffin | 60.8% |
| white soft paraffin | 29.4% |

The liquid paraffin and white soft paraffin are heated at 150°C for 1 hour and then cooled to 35 - 40°C. The lignocaine hydrochloride, sodium sulphacetamide, methyl and propyl hydroxybenzoates, and active substance are then added, in turn, to the resulting mixture, with 10 minutes' stirring after each addition. The final mixture is passed through a colloid mill and then stirred for 30 minutes.

Example 8 - Cream for treatment of dermatological conditions

| | |
|---|---|
| active substance | 5.0% |
| cetyl pyridinium chloride | 1.0% |
| cetostearyl alcohol | 10.8% |

| | |
|---|---|
| methyl hydroxybenzoate | 0.5% |
| propyl hydroxybenzoate | 10.1% |
| liquid paraffin | 10.1% |
| glycerol | 6.7% |
| silicone fluid 200/50 | 10.3% |
| water | to 100.0% |

The water is boiled and then allowed to cool to 80°C, after which the methyl hydroxybenzoate, propyl hydroxybenzoate, cetyl pyridinium chloride, and active substance are added to the water, and stirred until a clear solution is formed. The cetostearyl alcohol and liquid paraffin are stirred together while being heated until a homogeneous solution has formed, after which it is allowed to cool to 70 to 75°C. The aqueous solution is then carefully added to the paraffin solution, with constant stirring and slow cooling. The glycerin and silicone fluid are added to the resulting emulsion and stirred until a homogeneous mixture has formed.

Example 9 - Jelly for treatment of burns

| | |
|---|---|
| active substance | 2.0% |
| glycerine | 25.0% |
| methyl hydroxybenzoate | 0.5% |
| propyl hydroxybenzoate | 0.5% |
| sodium carboxymethylcellulose | 4.5% |
| lignocaine hydrochloride | 2.0% |
| water | to 100.0% |

The methyl hydroxybenzoate and propyl hydroxybenzoate are added, with stirring, to a portion of the water heated to boiling. When a clear solution has been formed, it is cooled and filtered. The glycerine is stirred at high speed while the sodium carboxymethylcellulose is added thereto, and stirring is continued until a smooth paste has been formed. The active substance and lignocaine hydrochloride are stirred in cold water until they have dissolved and the resulting solution is filtered.

The two aqueous solutions are mixed together and most of the remaining water required is added thereto. The pH is checked and adjusted as necessary to about 6.3 by adding normal sodium hydroxide solution or 10% hydrochloric acid. The solution is then added to the glycerine paste and adjusted to volume with further water. It is then stirred, initially slowly and then at high speed, for about 20 minutes.

A sample is then autoclaved, the viscosity is checked and adjusted, and the bulk is autoclaved, as described in Example 1.

Example 10 - Aqueous spray for treatment of burns and skin grafts

| | |
|---|---|
| active substance | 2.0% |
| methyl hydroxybenzoate | 0.5% |
| propyl hydroxybenzoate | 0.5% |
| water | 5.0% |
| buffered normal saline | to 100.0% |

A portion of the water is boiled and allowed to cool to 80$^{o}$C, and the methyl hydroxybenzoate and propyl hydroxybenzoate are added thereto and stirred

until dissolved. The active substance is dissolved in a second portion of the water. The two solutions are mixed and adjusted to volume. The pH is adjusted as necessary to between 6.0 and 6.5 by adding normal sodium hydroxide solution or 10% hydrochloric acid.

The solution is then filled into spray packs using, for example, Arctons as a propellant or, if the solution and the propellant are kept separate by means of a plastics membrane, using nitrogen or carbon dioxide, for example, as the propellant.

<u>Example 11</u> - Skin ointment for treatment of lacerations in humans and animals

| | |
|---|---|
| active substance | 2.0% |
| methyl hydroxybenzoate | 0.5% |
| propyl hydroxybenzoate | 0.5% |
| prednisolone | 0.3% |
| cod liver oil | 10.0% |
| chlorophyll | 2.0% |
| polyethylene glycol 4000 | 40.0% |
| propylene glycol | 45.0% |
| neoroli oil | up to 2.0% |
| abietis oil | up to 2.0% |

The prednisolone and half the polypropylene glycol are stirred together until the former has dissolved in the latter, and then the polyethylene glycol is added thereto, with stirring and gentle heating to a temperature not exceeding 55°C. The remainder of the polypropylene glycol is then added to the mixture, followed by the cod liver oil and then by the methyl hydroxybenzoate, propyl hydroxybenzoate and chlorophyll, with continuous stirring. The neoroli oil and abietis oil

are then added, and the mixture is allowed to cool, with intermittent stirring until the bulk begins to thicken and set.

Example 12 - Mouthwash

| | |
|---|---|
| active substance | 10.0% |
| methyl hydroxybenzoate | 0.5% |
| propyl hydroxybenzoate | 0.5% |
| sodium saccharin | 0.04% |
| flavouring | 0.5% |
| dye | 0.01% |
| sodium carboxymethylcellulose | 2.7% |
| water | to 100.0% |

A portion of water is heated to boiling and allowed to cool slightly, and then the methyl hydroxybenzoate and propyl hydroxybenzoate are added thereto, with stirring until a clear solution has formed. The dye, flavouring and saccharin are dissolved or well dispersed in a further portion of water. The active substance is dissolved in a third portion of water. The sodium carboxymethylcellulose is slowly added to a fourth portion of water stirred at high speed, and stirring is continued for about 15 minutes, until a fairly viscous gel has formed. The benzoate solution is then added to the gel, with stirring, followed by addition of the dye solution and then the active substance solution. Water is added to final volume, and stirring is continued for about 20 minutes.

Claims

1. A monomeric, dimeric or polymeric compound that is a condensation product of formaldehyde and allantoin in a molar proportion of 1 to at least 0.5, respectively, in which the monomeric compound or each monomeric unit is of the general formula

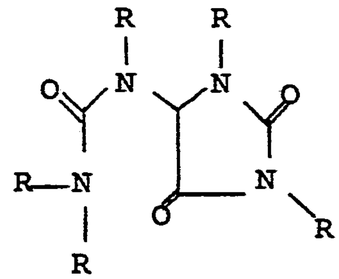

in which each R denotes H, $-CH_2OH$ or $(-CH_2-)_{1/2}$, with the provisos that

(a) in a monomeric compound, R cannot denote $(-CH_2-)_{1/2}$ and at least one R denotes $-CH_2OH$,

(b) in each unit of a dimeric compound and in each terminal unit of a polymeric compound, at least one symbol R denotes $(-CH_2-)_{1/2}$, and

(c) in each non-terminal unit of a polymeric compound, each of at least two symbols R denotes $(-CH_2-)_{1/2}$,

or a physiologically tolerable salt of such a compound, for use in the therapeutic treatment of humans or animals.

2. Imidazolidinyl urea or diazolidinyl urea, or a physiologically tolerable salt thereof, for use in the therapeutic treatment of humans or animals.

3. A compound as defined in claim 1 or claim 2, or a physiologically tolerable salt thereof, for use in the therapeutic treatment of diseases, malconditions and other disorders of the skin and

other membranes of the human or animal body.

4. A compound as defined in claim 1 or claim 2, or a physiologically tolerable salt thereof, for use in the therapeutic treatment of the intestines or uterus of an animal.

5. A compound as defined in claim 1 or claim 2, or a physiologically tolerable salt thereof, for use in the therapeutic treatment of animal hooves.

6. A compound as defined in claim 1 or claim 2, or a physiologically tolerable salt thereof, for use in the therapeutic treatment of the ears and eyes of humans or animals.

7. A compound as defined in claim 1 or claim 2, or a physiologically tolerable salt thereof, for the therapeutic treatment of cattle udders for the prevention or cure of mastitis.

8. A composition which comprises a therapeutically effective amcunt of a monomeric, dimeric or polymeric compound that is a condensation product of formaldehyde and allantoin in a molar proportion of 1 to at least 0.5, respectively, in which the monomeric compound or each monomeric unit is of the general formula

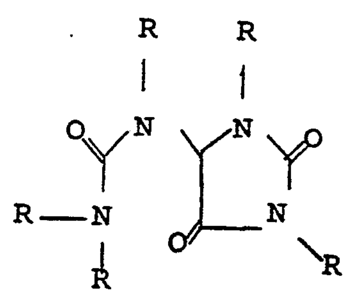

in which each R denotes H, $-CH_2OH$ or $(-CH_2-)_{1/2}$, with the provisos that

(a) in a monomeric compound, R cannot denote $(-CH_2-)_{1/2}$ and at least one R denotes $-CH_2OH$,

(b) in each unit of a dimeric compound and in each terminal unit of a polymeric compound, at least one symbol R denotes $(-CH_2-)_{1/2}$, and

(c) in each non-terminal unit of a polymeric compound, each of at least two symbols R denotes $(-CH_2-)_{1/2}$,

or a physiologically tolerable salt of such a compound, in admixture or conjunction with a pharmaceutically or veterinarily suitable carrier.

9. A composition as claimed in claim 8, wherein the said compound is imidazolidinyl urea.

10. A composition as claimed in claim 8, wherein the said compound is diazolidinyl urea.

11. A composition as claimed in any one of claims 8 to 10, formulated for external application to humans or animals.

12. A composition as claimed in any one of claims 8 to 10, formulated for internal application to humans or animals.

13. A composition as claimed in any one of claims 8 to 12, formulated in unit-dosage form.

14. A composition as claimed in any one of claims 8 to 10, formulated for internal application to humans or animals, and comprising the said compound or salt

in an amount of at least 1% by weight, based on the total weight of the composition, wherein the carrier comprises one or more carriers selected from (a) a non-liquid carrier, optionally in conjunction with a liquid carrier, (b) a liquid carrier other than water, optionally in conjunction with water, and (c) water in conjunction with another substance.

15. A composition as claimed in claim 14, wherein the said amount is at least 1.5% by weight.

16. A composition as claimed in any one of claims 8 to 10, formulated for external application to humans or animals and not being a cosmetic composition, and comprising the said compound or salt in an amount of at least 1% by weight, based on the total weight of the composition, in admixture or conjunction with one or more carriers selected from (a) a non-liquid carrier, optionally in conjunction with a liquid carrier (b) a liquid carrier in conjunction with a propellant, (c) a liquid carrier other than water, optionally in conjunction with water, and (d) water in conjunction with another substance.

17. A composition as claimed in any one of claims 8 to 10, formulated for external application to humans or animals and comprising the said compound or salt in an amount of at least 1.5% by weight, based on the total weight of the composition, in admixture or conjunction with one or more carriers selected from (a) a non-liquid carrier, optionally in conjunction with a liquid carrier, (b) a liquid carrier in conjunction with a propellant, (c) a liquid carrier other than water, optionally in conjunction with water, and (d) water in conjunction with another substance.

18. A composition as claimed in any one of claims 15 to 17, wherein the said amount is at least 2% by weight.

19. A composition as claimed in any one of claims 15 to 18, wherein the said amount is at least 5% by weight.

20. A composition as claimed in any one of claims 8 to 19, wherein the carrier comprises physiologically tolerable saline solution.

21. A composition as claimed in any one of claims 8 to 10, in the form of a bolus, capsule, pill, tablet or the like for oral administration to animals.

22. A composition as claimed in claim 21, wherein the said compound or salt is present in an amount within the range of from 2.5 to 15% by weight, based on the total weight of the composition (excluding, in the case of a capsule, the shell).

23. A composition as claimed in any one of claims 8 to 10, in a form suitable for uterine infusion in animals and comprising the said compound or salt in admixture or conjunction with physiologically tolerable saline solution.

24. A composition as claimed in claim 23, wherein the said compound or salt is present in an amount within the range of from 2 to 10% by weight, based on the total weight of the composition.

25. A composition as claimed in any one of claims 8 to 10, in a form suitable for external application to humans or animals by spraying, and comprising the said compound or salt in admixture or conjunction with physiologically tolerable saline solution.

26. A composition as claimed in claim 25, wherein the said compound or salt is present in an amount within the range of from 1 to 10% by weight, based on the total weight of the composition.

27. A composition as claimed in any one of claims 8 to 10, in a form suitable for external application to humans or animals by spraying, and comprising the said compound or salt in admixture or conjunction with a film-forming agent (other than the said compound and allantoin) and a volatile diluent.

28. A composition as claimed in claim 27, wherein the said compound or salt is present in an amount within the range of from 5 to 30% by weight, based on the total weight of the composition.

29. A composition as claimed in any one of claims 8 to 10, in the form of a gel or the like for intramammary administration to cattle.

30. A composition as claimed in claim 29, wherein the said compound or salt is present in an amount within the range of from 1 to 10% by weight, based on the total weight of the composition.

31. A composition as claimed in any one of claims 8 to 10, in the form of a gel, jelly, cream, ointment, wax, or the like suitable for extramammary application

to cattle and comprising the said compound or salt in an amount of at least 5% by weight, based on the total weight of the composition.

32. A composition as claimed in claim 31, wherein the said compound or salt is present in an amount within the range of from 5 to 10% by weight, based on the total weight of the composition.

33. A composition as claimed in any one of claims 8 to 10, in the form of an ointment.

34. A composition as claimed in claim 33, wherein the ointment is suitable for application to eyes or ears of humans or animals.

35. A composition as claimed in claim 33 or claim 34, wherein the said compound or salt is present in an amount within the range of from 1 to 10% by weight, based on the total weight of the composition.

36. A composition as claimed in any one of claims 8 to 10, in a form suitable for external application to humans or animals, wherein the composition comprises the said compound or salt in an amount of more than 1% by weight, based on the total weight of the composition, and additionally comprises a topical anaesthetic agent.

37. A composition as claimed in claim 36, in the form of a gel, jelly, cream, ointment or the like.

38. A composition as claimed in claim 36 or claim 37, wherein the said compound or salt is present in an amount within the range of from 1 to 20% by weight, based on the total weight of the composition.

39. A composition as claimed in any one of claims 8 to 10, in the form of a gel, jelly, cream, ointment or the like and not being a cosmetic composition, and comprising the said compound or salt in an amount of at least 1% by weight, based on the total weight of the composition.

40. A composition as claimed in any one of claims 8 to 10, in the form of a gel, jelly, cream, ointment or the like and comprising the said compound or salt in an amount of at least 1.5% by weight, based on the total weight of the composition.

41. A composition as claimed in claim 39 or claim 40, wherein the said amount is at least 2% by weight, based on the total weight of the composition.

42. A composition as claimed in claim 39 or claim 40, wherein the said amount is at least 5% by weight, based on the total weight of the composition.

43. A composition as claimed in any one of claims 8 to 10, in the form of a mouthwash, wherein the composition additionally comprises one or more auxiliaries selected from a sweetening agent, a flavouring agent, and a colouring agent.

44. A composition as claimed in claim 43, wherein the said compound or salt is present in an amount within the range of from 2 to 15% by weight, based on the total weight of the composition.

45. A composition as claimed in any one of claims 8 to 10, in a form suitable for external application to humans and animals, wherein the composition

comprises at least 10% by weight of an alcohol, based on the total weight of the composition.

46. A composition as claimed in claim 45, wherein the said compound or salt is present in an amount within the range of from 1 to 10% by weight, based on the total weight of the composition.

47. A surgical dressing, surgical swab or the like that is impregnated with a compound as defined in claim 1 or claim 2, or a physiologically tolerable salt thereof.

48. A process for the treatment of a surgical dressing, surgical swab or the like which comprises applying to it a compound as defined in claim 1 or claim 2, or a physiologically tolerable salt thereof.

49. A process as claimed in claim 48, wherein the said compound or salt is applied in the form of a powder.

50. A process as claimed in claim 48, wherein the said compound or salt is applied in the form of a solution.

51. A process as claimed in any one of claims 48 to 50, wherein the dressing, swab or the like is impregnated with the said compound or salt.

52. A process for the treatment of a contact lens, which comprises treating it with a solution of a compound as defined in claim 1 or claim 2, or a physiologically tolerable salt thereof.

53. A composition substantially as described in any one of Examples 1 to 12 herein.

0080281

European Patent Office

# EUROPEAN SEARCH REPORT

Application number

EP 82 30 5875

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | US-A-4 271 176 (BERKE-ROSEN) *Columns 1,4* --- | 1 | C 07 D 233/78<br>C 07 D 233/72<br>A 61 K 31/415 |
| D,X | DE-A-2 304 156 (BEECHAM) *Pages 2-4,7* --- | 1-4,8-9,11,25 | |
| D,X | US-A-3 248 285 (BERKE) *Columns 1-6* ----- | 1-3,6,8-11 | |
| | | | TECHNICAL FIELDS SEARCHED (Int. Cl. 3) |
| | | | C 07 D 233/00<br>A 61 K 31/00 |

The present search report has been drawn up for all claims

| Place of search | Date of completion of the search | Examiner |
|---|---|---|
| THE HAGUE | 08-02-1983 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82